# EUROPEAN PATENT APPLICATION

(11) **EP 3 321 047 A1**
(43) Date of publication of application: **16.05.2018**
(21) Application number: 15897638.1
(22) Date of filing: 09.07.2015
(51) Int. Cl.: B25J 17/02, B25J 18/06

(54) **SURGICAL ROBOT**

(71) Applicant: Kawasaki Jukogyo Kabushiki Kaisha, Kobe-shi, Hyogo 650-8670 (JP)
(72) Inventor: KAN, Kazutoshi, Hyogo 673-8666 (JP)
(74) Representative: Witte, Weller & Partner Patentanwälte mbB
(86) International application number: PCT/JP2015/003486
(87) International publication number: WO 2017/006375

(57) **Abstract**

The present invention includes: a robot main body (2) including a hollow flexible shaft (25) having flexibility, a hollow bending joint (27, 28) including a proximal end (27a) continuous with a distal end (25b) of the flexible shaft, the bending joint being bendable in a direction perpendicular to an axis of the bending joint, a wrist joint (30) continuous with a distal end (28b) of the bending joint and rotatable around an axis of the wrist joint, an end effector (22) attached to the wrist joint, a hollow torque transmission tube(44) having flexibility, inserted through the flexible shaft and the bending joint, and including a distal end (44b) attached to the wrist joint, and a bending joint operating cable (41, 42) inserted through the flexible shaft and including a distal end (43a) attached to the bending joint, the bending joint being configured to perform a bending operation by operating the bending joint operating cable in an extending direction of the bending joint operating cable; a guide pipe (6) having flexibility, the flexible shaft being inserted through the guide pipe; and a robot main body driving mechanism (51) including a bending joint driving portion (35, 36) configured to operate the bending joint operating cable in the extending direction of the bending joint operating cable by driving force of the bending joint driving portion and a wrist joint driving portion (38) configured to rotate a proximal end of the torque transmission tube by driving force of the wrist joint driving portion.

## Description

### Technical Field

The present invention relates to a surgical robot.

### Background Art

A medical system including a treatment tool inserted into a body of a patient has been known (see PTL 1, for example).

The medical system includes an insertion portion inserted into a body of a patient. The insertion portion includes: a curved portion; and right and left arms attached to a tip end of the curved portion. Each of the arms can be curved in a direction perpendicular to an axis of the arm. An elongated sheath portion having flexibility and a treatment tool provided at a tip end portion of the sheath portion are inserted through each arm, and the treatment tool projects from a tip end opening of the arm.

### Citation List

### Patent Literature

PTL 1: International Publication No. 2014/156286, Description

### Summary of Invention

### Technical Problem

According to the medical system described in PTL 1, a direction of the treatment tool is changed in such a manner that the arm curves a root of the sheath portion projecting from the tip end of the curved portion. Therefore, there is a problem that as a projection amount of the treatment tool from the tip end of the arm increases, it becomes difficult to accurately set the direction of the treatment tool to a target direction.

### Solution to Problem

To solve the above problem, a surgical robot according to one aspect of the present invention includes: a robot main body including a hollow flexible shaft having flexibility, a hollow bending joint including a proximal end continuous with a distal end of the flexible shaft, the bending joint being bendable in a direction perpendicular to an axis of the bending joint, a wrist joint continuous with a distal end of the bending joint and rotatable around an axis of the wrist joint, an end effector attached to the wrist joint, a hollow torque transmission tube having flexibility, inserted through the flexible shaft and the bending joint, and including a distal end attached to the wrist joint, and a bending joint operating cable inserted through the flexible shaft and including a distal end attached to the bending joint, the bending joint being configured to perform a bending operation by operating the bending joint operating cable in an extending direction of the bending joint operating cable; a guide pipe having flexibility, the flexible shaft being inserted through the guide pipe; and a robot main body driving mechanism including a bending joint driving portion configured to operate the bending joint operating cable in the extending direction of the bending joint operating cable by driving force of the bending joint driving portion and a wrist joint driving portion configured to rotate a proximal end of the torque transmission tube by driving force of the wrist joint driving portion.

According to this configuration, the direction of the end effector can be changed by the bending joint continuous with the distal end of the flexible shaft. Therefore, the end effector can be directed in a target direction in the vicinity of a treated part. On this account, the end effector can be accurately directed in the target direction.

Further, the operator can easily predict the movement of the end effector by the bending of the bending joint.

Further, an angular position of the end effector directed in the target direction can be adjusted around an axis of the end effector by rotating the wrist joint.

The wrist joint is rotated by rotating the torque transmission tube. Therefore, it is possible to prevent a case where the wrist joint moves when the flexible shaft and the bending joint are bent.

Thus, operability of the surgical robot can be improved.

The guide pipe may include a distal end curving mechanism configured to curve a distal end of the guide pipe.

According to this configuration, it is possible to prevent a case where the surgical robot in operation interferes with a different surgical robot.

Rigidity of the guide pipe in a bending direction may be higher than rigidity of the flexible shaft in the bending direction.

According to this configuration, it is possible to prevent a case where the guide pipe deforms when the flexible shaft is rotated around the axis thereof. Thus, an angular position of the flexible shaft can be changed while maintaining an extending direction of the flexible shaft.

The surgical robot may be configured such that: the robot main body includes an end effector operating cable inserted through the torque transmission tube and including a distal end attached to the end effector; the end effector is configured to be operated by operating the end effector operating cable in an extending direction of the end effector operating cable; and the robot main body driving mechanism includes an end effector driving portion configured to operate the end effector operating cable in the extending direction of the end effector operating cable by driving force of the end effector driving portion.

According to this configuration, it is possible to prevent a case where by bending the bending joint, the end effector operating cable is moved in an extending direction, and this operates the end effector.

### Advantageous Effects of Invention

The present invention has an effect of being able to improve the operability of the surgical robot.

### Brief Description of Drawings

Fig. 1 is a diagram schematically showing a configuration example of a surgical robot system including a surgical robot according to an embodiment of the present invention.
Fig. 2 is a diagram showing a configuration example of the surgical robot of Fig. 1.
Fig. 3 is a perspective view showing a configuration example of a distal end of a robot main body of the surgical robot of Fig. 1.
Fig. 4 is a diagram schematically showing a configuration example of the robot main body of the surgical robot of Fig. 1.
Fig. 5A is a diagram showing a configuration example of the distal end of the robot main body of the surgical robot of Fig. 1 and is a diagram showing a state where a joint portion of the robot main body is linearly stretched.
Fig. 5B is a diagram showing a configuration example of the distal end of the robot main body of the surgical robot of Fig. 1 and is a diagram showing a state where the joint portion of the robot main body is bent.
Fig. 6 is a partial breakaway view showing a configuration example of a wrist joint of the robot main body of the surgical robot of Fig. 1.
Fig. 7A is a diagram showing a configuration example of the distal end of the robot main body of the surgical robot of Fig. 1 and is a diagram showing a configuration example of a first bending joint operating cable.
Fig. 7B is a diagram showing a configuration example of the distal end of the robot main body of the surgical robot of Fig. 1 and is a diagram showing a configuration example of a second bending joint operating cable.
Fig. 8A is a B-B arrow view showing a configuration example of the distal end of the robot main body of the surgical robot of Fig. 1.
Fig. 8B is a C-C arrow view showing a configuration example of the distal end of the robot main body of the surgical robot of Fig. 1.
Fig. 9 is a block diagram schematically showing a configuration example of a control system of the surgical robot of Fig. 1.

### Description of Embodiments

Hereinafter, an embodiment of the present invention will be explained in reference to the drawings. It should be noted that the present invention is not limited by the present embodiment. In the following explanations and the drawings, the same reference signs are used for the same or corresponding components, and a repetition of the same explanation is avoided.

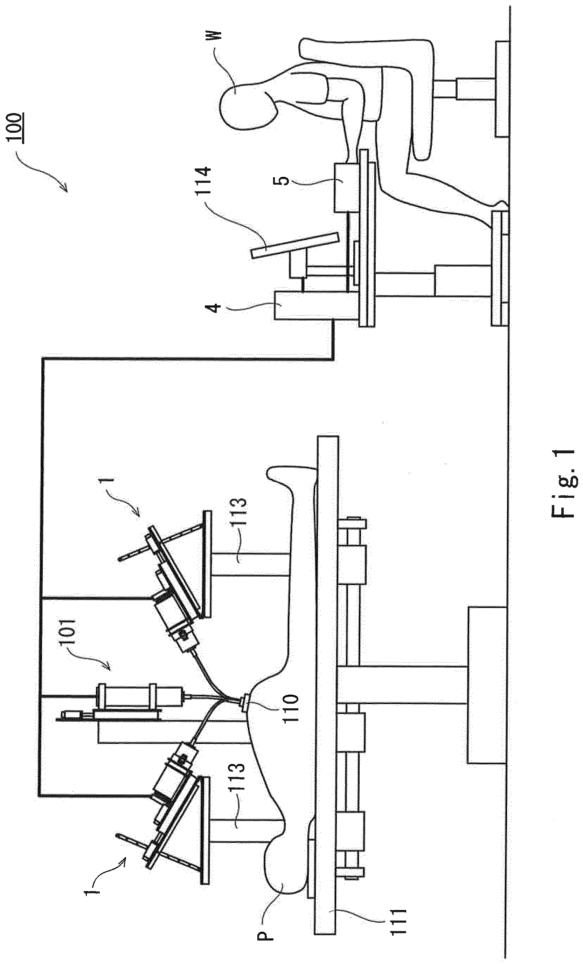

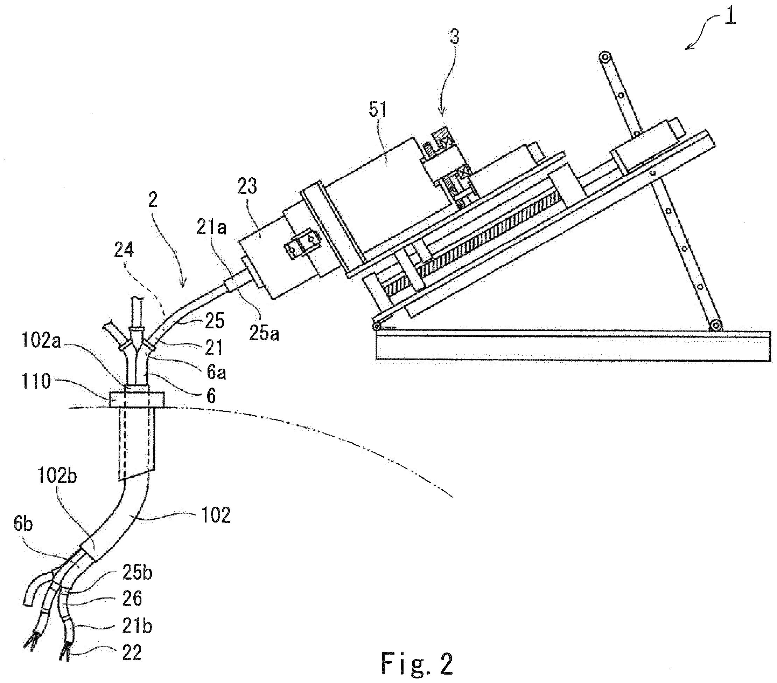

Fig. 1 is a diagram schematically showing a configuration example of a surgical robot system 100 including a surgical robot 1 according to the embodiment of the present invention. Fig. 2 is a diagram showing a configuration example of the surgical robot 1.

As shown in Fig. 1, the surgical robot system 100 is a system used when an operator W remotely operates a surgical tool from an outside to perform minimally invasive surgery, the surgical tool being provided at a distal end of the surgical robot 1 and inserted into a body of a patient P on an operating table 111.

For example, the surgical robot system 100 includes one or more surgical robots 1 and an endoscope 101.

The surgical robot 1 is supported by a surgical robot support base 113 attached to the operating table 111. The surgical robot 1 includes an arm formed in a thin and long shape and further includes a surgical tool at a distal end of the arm. A treated part in the body of the patient P is treated by the surgical tool. In the present embodiment, the surgical robot 1 is a robot including a pair of forceps at the distal end of the arm. However, the surgical tool at the distal end of the arm is not limited to the forceps, and various surgical tools are applicable.

The endoscope 101 is used by the operator W to visually recognize the inside of the body of the patient P and includes a video camera and a light at a distal end of the endoscope 101. An image taken by the video camera of the endoscope 101 is displayed on a display device 114. With this, the operator W can operate the surgical robot 1 to perform surgery while visually recognizing states of the distal end of the arm and the surgical tool in the body of the patient P and a state of the treated part.

As shown in Fig. 2, the surgical robots 1 are inserted into a collectively bundling pipe 102 to be collectively bundled. The collectively bundling pipe 102 has flexibility and is formed in a hollow tubular shape.

### Configuration Example of Robot Main Body

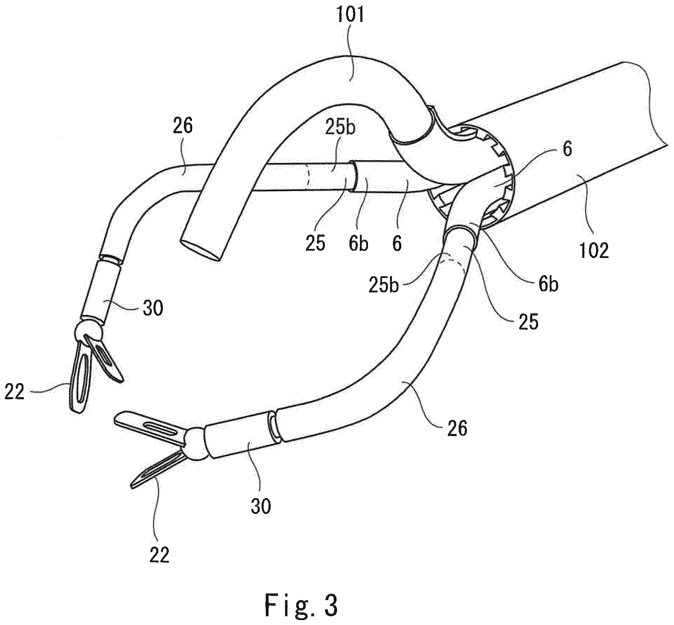

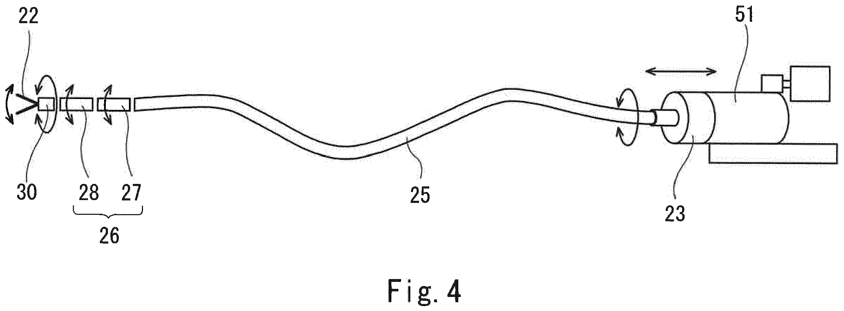

Fig. 3 is a perspective view showing a configuration example of a distal end of a robot main body 2 of the surgical robot 1. Fig. 4 is a diagram schematically showing a configuration example of the robot main body 2.

As shown in Fig. 2, the surgical robot 1 includes the robot main body 2, a driving portion 3, a control unit 4 (see Fig. 1), an operating portion 5 (see Fig. 1), and a guide pipe 6.

As shown in Fig. 2, the robot main body 2 includes an arm 21, a pair of forceps (end effector) 22 provided at a distal end 21b of the arm 21, and a driving force transmission mechanism 24. Further, the robot main body 2 includes a base 23. The base 23 is configured to be attachable to the driving portion 3. By attaching the base 23 to the driving portion 3, the robot main body 2 can be coupled to the driving portion 3.

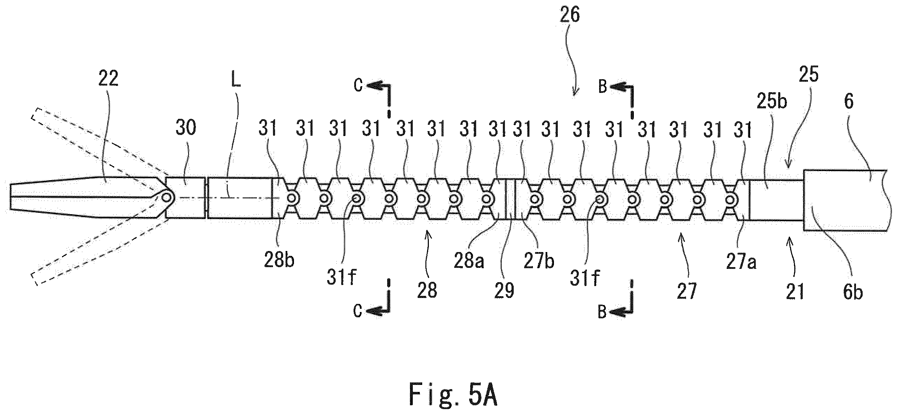

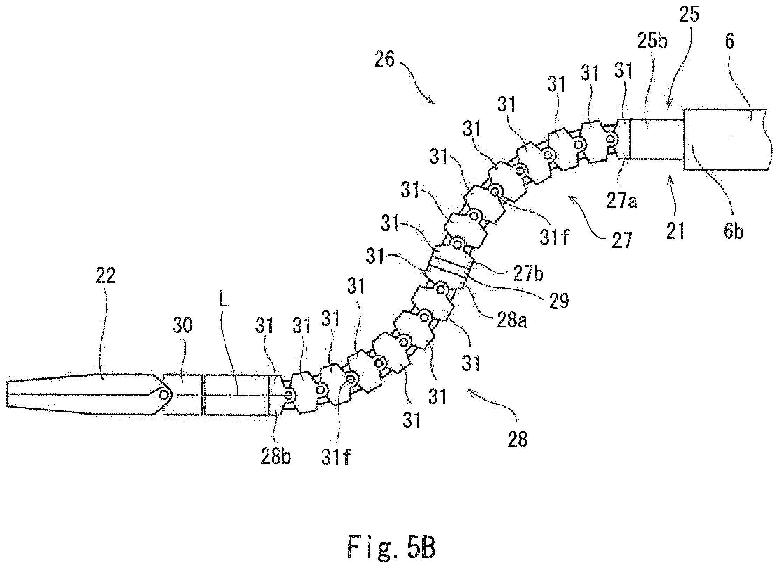

Fig. 5A is a diagram showing a configuration example of the distal end of the robot main body 2 and is a diagram showing a state where a joint portion 26 of the robot main body 2 is linearly stretched. Fig. 5B is a diagram showing a configuration example of the distal end of the robot main body 2 and a diagram showing a state where the joint portion 26 of the robot main body 2 is bent.

As shown in Figs. 5A and 5B, the arm 21 includes a hollow flexible shaft 25 having flexibility and the joint portion 26.

The flexible shaft 25 is, for example, a tubular body. As shown in Fig. 2, a proximal end 25a of the flexible shaft 25 is attached and fixed to the base 23.

The flexible shaft 25 has flexibility in a bending direction and high rigidity in an axial direction. Further, the flexible shaft 25 has rigidity to rotational torque around an axis.

As shown in Figs. 5A and 5B, a proximal end of the joint portion 26 (i.e., a proximal end 27a of a first bending joint 27) is continuous with a distal end 25b of the flexible shaft 25. The joint portion 26 is a hollow tubular body, and an internal space of the joint portion 26 communicates with an internal space of the flexible shaft 25.

The joint portion 26 includes the first bending joint 27, a second bending joint 28, a connecting portion 29, and a wrist joint 30. The first bending joint 27, the second bending joint 28, the connecting portion 29, and the wrist joint 30 are arranged on the same axis. An outer peripheral surface of the joint portion 26 is covered with a cover (not shown), and the joint portion 26 is substantially the same in diameter as the flexible shaft 25.

The first bending joint 27 is a hollow tubular body, and the proximal end 27a thereof is attached to the distal end 25b of the flexible shaft 25 so as to be continuous with the distal end 25b. It should be noted that the term "continuous" denotes not only a case where two members are directly connected to each other but also a case where two members are indirectly connected to each other with another member interposed therebetween.

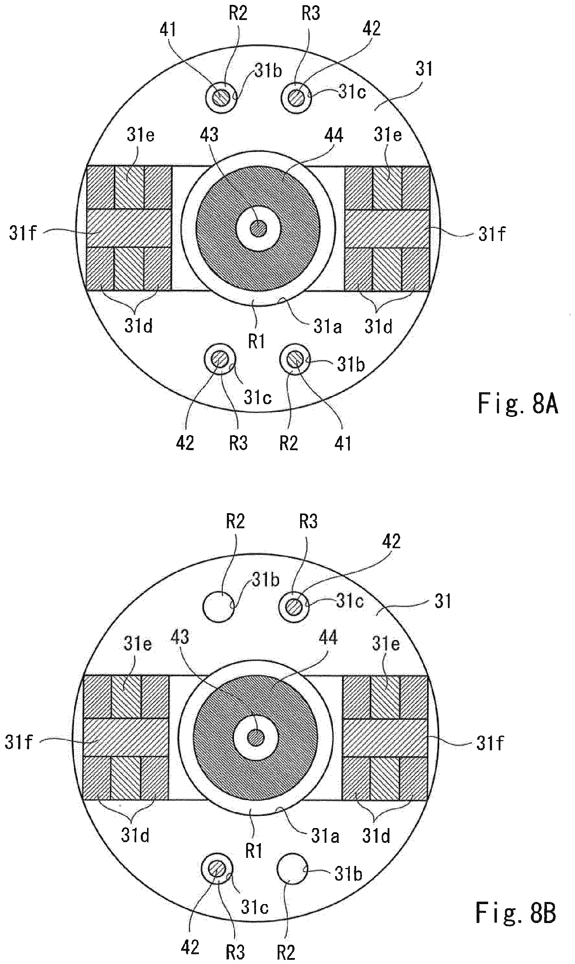

Fig. 8A is a B-B arrow view showing a configuration example of the distal end of the robot main body 2. Fig. 8B is a C-C arrow view showing a configuration example of the distal end of the robot main body 2.

The first bending joint 27 includes a plurality of frame members 31 that are continuous in a row in an axial direction of the joint portion 26. Each of the frame members 31 is formed in a columnar shape extending in the axial direction of the joint portion 26. The frame member 31 is formed in such a tapered shape that when viewed from a direction perpendicular to an axis of the frame member 31 and a bending direction of the below-described first bending joint 27 (i.e., when viewed from an extending direction of a below-described pin 31f), a thickness of the frame member 31 in an axial direction decreases as the frame member 31 extends away from the axis of the frame member 31. To be specific, the frame member 31 is formed so as to be thinner as it extends upward and downward in Fig. 5A. With this, interference between opposing end surfaces of the adjacent frame members 31 when the first bending joint 27 is bent is avoided.

As shown in Figs. 8A and 8B, the frame member 31 includes a first insertion hole 31a, a pair of second insertion holes 31b, and a pair of third insertion holes 31c.

The first insertion hole 31a is formed on the axis of the frame member 31, and a below-described torque transmission tube 44 is inserted through the first insertion hole 31a. The first insertion holes 31a of the plurality of frame members 31 that are continuous in a row constitute a first route R1 extending in an extending direction of the arm 21.

The pair of second insertion holes 31b connect both end surfaces of the frame member 31 and extend parallel to the axis of the frame member 31. When viewed from the direction perpendicular to the axis of the frame member 31 and the bending direction of the below-described first bending joint 27 (i.e., when viewed from the extending direction of the below-described pin 31f), one of the pair of second insertion holes 31b is located at an opposite side of the other of the pair of second insertion holes 31b across the axis of the frame member 31. To be specific, in Fig. 5A, one of the pair of second insertion holes 31b is formed above the below-described pin 31f, and the other is formed under the below-described pin 31f. Both end portions of a below-described first bending joint operating cable 41 are inserted through the respective second insertion holes 31b. The pairs of second insertion holes 31b of the plurality of frame members 31 that are continuous in a row constitute a pair of second routes R2 extending in the extending direction of the arm 21. Therefore, when viewed from the extending direction of the below-described pin 31f, one of the pair of second routes R2 is located at an opposite side of the other of the pair of second routes R2 across the axis of the frame member 31.

The pair of third insertion holes 31c connect both end surfaces of the frame member 31 and extend parallel to the axis of the frame member 31. When viewed from the direction perpendicular to the axis of the frame member 31 and the bending direction of the below-described first bending joint 27, one of the pair of third insertion holes 31c is located at an opposite side of the other of the pair of third insertion holes 31c across the axis of the frame member 31. To be specific, in Fig. 5A, one of the pair of third insertion holes 31c is formed above the below-described pin 31f, and the other is formed under the below-described pin 31f. Both end portions of a below-described second bending joint operating cable 42 are inserted through the respective third insertion holes 31c. The pairs of third insertion holes 31c of the plurality of frame members 31 that are continuous in a row constitute a pair of third routes R3 extending in the extending direction of the arm 21. Therefore, when viewed from the extending direction of the below-described pin 31f, one of the pair of third routes R3 is located at an opposite side of the other of the pair of third routes R3 across the axis of the frame member 31.

A pair of first projecting portions 31d are formed to project from one of the end surfaces of the frame member 31 outward in an extending direction of the frame member 31, and a pair of second projecting portions 31e are formed to project from the other end surface of the frame member 31 outward in the extending direction of the frame member 31. The pair of first projecting portions 31d of the frame member 31 and the pair of second projecting portions 31e of the adjacent frame member 31 are coupled to each other by a pair of pins 31f lined up on the same straight line. With this, each frame member 31 is coupled to the adjacent frame member 31 so as to be swingable about an axis (swing axis) of the pair of pins 31f. The swing axes of the frame members 31 are parallel to one another, and the first bending joint 27 is configured to be bendable such that a distal end 27b of the first bending joint 27 turns toward a direction (hereinafter also referred to as the bending direction) perpendicular to the axis of the frame member 31 and the swing axis. In Fig. 5A, the axis of the frame member 31 denotes an axis extending in a paper surface leftward/rightward direction, and the swing axis denotes an axis extending in a paper surface depth direction.

As described above, when viewed from the extending direction of the below-described pin 31f, one of the pair of second routes R2 is located at an opposite side of the other of the pair of second routes R2 across the axis of the frame member 31. Therefore, when the first bending joint 27 performs the bending operation, the route length of the second route R2 located at a bending-direction inner side out of the pair of second routes R2 becomes short, and the route length of the second route R2 located at a bending-direction outer side becomes long. Similarly, when viewed from the extending direction of the below-described pin 31f, one of the pair of third routes R3 is located at an opposite side of the other of the pair of third routes R3 across the axis of the frame member 31. Therefore, when the first bending joint 27 performs the bending operation, the route length of the third route R3 located at the bending-direction inner side out of the pair of third routes R3 becomes short, and the route length of the third route R3 located at the bending-direction outer side becomes long.

Since the second bending joint 28 is the same in configuration as the first bending joint 27, an explanation thereof is omitted.

The connecting portion 29 is a hollow tubular body and connects the first bending joint 27 and the second bending joint 28.

The wrist joint 30 rotates the forceps 22 around an axis L of the distal end 21b of the arm 21. The wrist joint 30 is a plate-shaped body extending on a plane perpendicular to an axis of the arm 21 (axis of the joint portion 26) and is provided with a through hole 30a at a center portion of the wrist joint 30, the through hole 30a connecting a proximal end surface of the wrist joint 30 and a distal end surface of the wrist joint 30. The through hole 30a is a hole through which a below-described forceps operating cable 43 is inserted. The through hole 30a is formed on an axis of the distal end 21b of the arm 21. The wrist joint 30 is attached to a distal end 28b of the second bending joint 28 through a bearing (not shown) so as to be continuous with the distal end 28b. Therefore, the wrist joint 30 is configured to be rotatable around an axis of the wrist joint 30 (around the axis L of the distal end 21b of the arm 21) relative to the flexible shaft 25, the first bending joint 27, and the second bending joint 28.

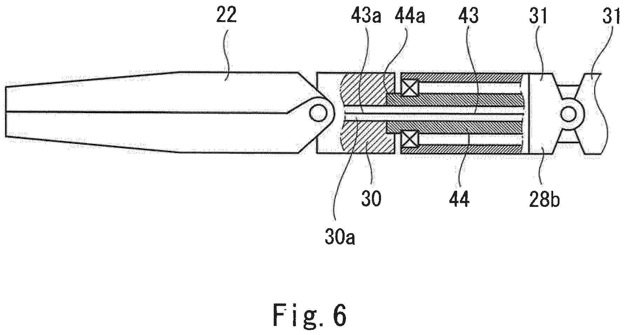

A distal end 44b of the below-described torque transmission tube 44 is fixed to the proximal end surface of the wrist joint 30, i.e., a peripheral portion of the through hole 30a (see Fig. 6).

The end effector is a surgical tool. In the present embodiment, the end effector is the forceps 22. The forceps 22 is attached to the distal end surface of the wrist joint 30. To be specific, the forceps 22 is continuous with a distal end of the joint portion 26 (i.e., the distal end 28b of the second bending joint 28).

The forceps 22 includes an opening/closing operation operating mechanism (not shown) including an operating cable coupling portion. The operating cable coupling portion is a portion to which a distal end 43a of the below-described forceps operating cable 43 is coupled. The opening/closing operation operating mechanism of the forceps 22 is a mechanism configured to, when the operating cable coupling portion is moved in a predetermined direction, open or close the forceps by a predetermined amount in accordance with a movement distance of the operating cable coupling portion. The operating cable coupling portion is biased by a biasing mechanism (not shown) in a direction from a proximal end of the forceps operating cable 43 toward the distal end 43a. With this, when the forceps operating cable 43 is pulled in a direction from the distal end 43a to the proximal end, the operating cable coupling portion is moved in a movement direction of the distal end 43a of the forceps operating cable 43 against the biasing force of the biasing mechanism. Thus, the forceps 22 performs, for example, a closing operation to perform a holding operation of a target. Further, when the forceps operating cable 43 is sent out in a direction from the proximal end to the distal end 43a, the forceps operating cable 43 is slackened. However, the biasing mechanism moves the operating cable coupling portion in a direction opposite to the movement direction of the distal end 43a of the forceps operating cable 43 so as to absorb the slackening of the forceps operating cable 43. Thus, the forceps 22 performs, for example, an opening operation to perform a releasing operation of the target.

As above, an internal space from a proximal end 21a of the arm 21 to the distal end 21b is a communicating space, and the first bending joint operating cable 41, the second bending joint operating cable 42, the forceps operating cable 43, and the torque transmission tube 44 in the below-described driving force transmission mechanism 24 are inserted through this internal space.

The driving force transmission mechanism 24 is a mechanism configured to transmit driving force of a below-described robot main body driving mechanism 51 of the driving portion 3 to mechanisms that are continuous with the distal end 25b of the flexible shaft 25. To be specific, the driving force transmission mechanism 24 is a mechanism configured to: connect between the robot main body driving mechanism 51 and the first bending joint 27, between the robot main body driving mechanism 51 and the second bending joint 28, between the robot main body driving mechanism 51 and the wrist joint 30, and between the robot main body driving mechanism 51 and the forceps 22; and transmit the driving force of the robot main body driving mechanism 51 to the first bending joint 27, the second bending joint 28, the wrist joint 30, and the forceps 22. As shown in Figs. 3 and 4, the driving force transmission mechanism 24 includes the first bending joint operating cable 41, a first bending joint operating cable operating portion (not shown), the second bending joint operating cable 42, a second bending joint operating cable operating portion (not shown), the forceps operating cable 43, a forceps operating cable operating portion (not shown), the torque transmission tube 44, and a torque transmission tube rotating portion (not shown).

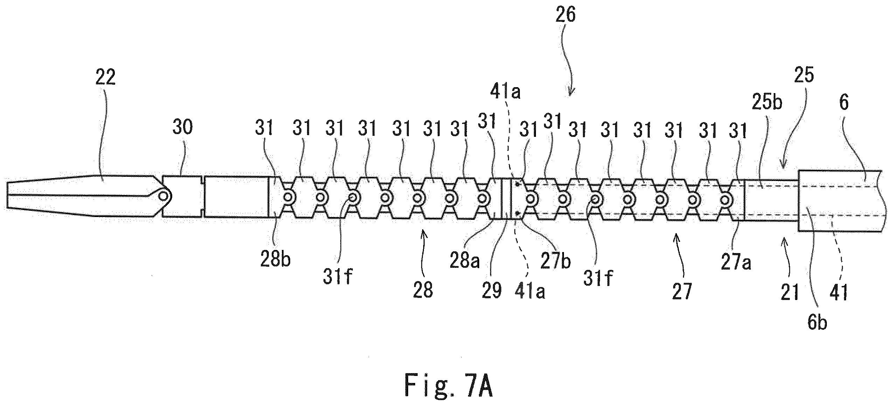

Fig. 7A is a diagram showing a configuration example of the distal end of the robot main body 2 and is a diagram showing a configuration example of the first bending joint operating cable 41.

As shown in Fig. 7A, both end portions 41a of the first bending joint operating cable 41 are fixed to the frame member 31 located at the distal end 27b of the first bending joint 27.

Apart of the first bending joint operating cable 41 which part extends from one of the end portions 41a to an intermediate portion of the first bending joint operating cable 41 extends through an internal space of one of the pair of second routes R2 of the first bending joint 27 and the internal space of the flexible shaft 25 to reach an internal space of the base 23. Further, a part of the first bending joint operating cable 41 which part extends from the other end portion 41a to the intermediate portion extends through an internal space of the other of the pair of second routes R2 of the first bending joint 27 and the internal space of the flexible shaft 25 to reach the internal space of the base 23.

The first bending joint operating cable operating portion is a mechanism provided in the base 23 and configured to move the intermediate portion of the first bending joint operating cable 41 in an extending direction of the first bending joint operating cable 41 by the driving force of the driving portion 3, the intermediate portion being located in the internal space of the base 23. When the intermediate portion of the first bending joint operating cable 41 is moved toward one side in the extending direction of the first bending joint operating cable 41 by the driving force of the driving portion 3, a part of the first bending joint operating cable 41 which part extends from the intermediate portion to one of the end portions 41a is pulled, and the one end portion 41a moves toward the proximal end 21a of the arm 21. With this, the route length of one of the pair of second routes R2 of the first bending joint 27 becomes short, the one second route R2 being a route through which the part of the first bending joint operating cable 41 which part extends from the intermediate portion to the one end portion 41a extends. Thus, the first bending joint 27 performs the bending operation of bending toward the one second route R2. Further, a part of the first bending joint operating cable 41 which part extends from the intermediate portion to the other end portion 41a is sent out to be sent into the other second route R2 which is increased in the route length out of the pair of second routes R2.

On the other hand, when the intermediate portion of the first bending joint operating cable 41 is moved toward the other side in the extending direction of the first bending joint operating cable 41 by the driving force of the driving portion 3, the part of the first bending joint operating cable 41 which part extends from the intermediate portion to the other end portion 41a is pulled, and the other end portion 41a moves toward the proximal end 21a of the arm 21. With this, the route length of the other of the pair of second routes R2 of the first bending joint 27 becomes short, the other second route R2 being a route through which the part of the first bending joint operating cable 41 which part extends from the intermediate portion to the other end portion 41a extends. Thus, the first bending joint 27 performs the bending operation of bending toward the other second route R2. Further, the part of the first bending joint operating cable 41 which part extends from the intermediate portion to the one end portion 41a is sent out to be sent into the one second route R2 which is increased in the route length out of the pair of second routes R2.

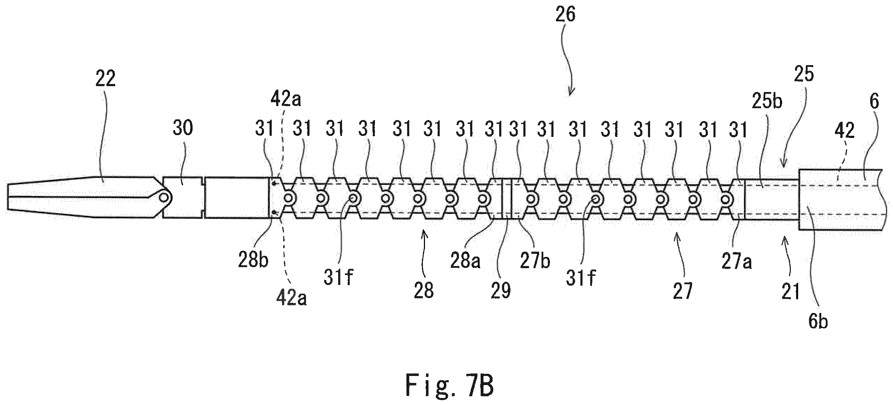

Fig. 7B is a diagram showing a configuration example of the distal end of the robot main body 2 and is a diagram showing a configuration example of the second bending joint operating cable 42.

As shown in Fig. 7B, both end portions 42a of the second bending joint operating cable 42 are fixed to the frame member 31 located at the distal end 28b of the second bending joint 28. A part of the second bending joint operating cable 42 which part extends from one of the end portions 42a to an intermediate portion of the second bending joint operating cable 42 extends through an internal space of one of the pair of third routes R3 of the second bending joint 28, an internal space of the connecting portion 29, an internal space of one of the pair of third routes R3 of the first bending joint 27, and the internal space of the flexible shaft 25 to reach the internal space of the base 23. Further, a part of the second bending joint operating cable 42 which part extends from the other end portion 42a to an intermediate portion of the second bending joint operating cable 42 extends through an internal space of the other of the pair of third routes R3 of the second bending joint 28, the internal space of the connecting portion 29, an internal space of the other of the pair of third routes R3 of the first bending joint 27, and the internal space of the flexible shaft 25 to reach the internal space of the base 23.

The second bending joint operating cable operating portion is a mechanism provided in the base 23 and configured to move the intermediate portion of the second bending joint operating cable 42 in an extending direction of the second bending joint operating cable 42 by the driving force of the driving portion 3, the intermediate portion being located in the internal space of the base 23. When the intermediate portion of the second bending joint operating cable 42 is moved toward one side in the extending direction of the second bending joint operating cable 42 by the driving force of the driving portion 3, a part of the second bending joint operating cable 42 which part extends from the intermediate portion to one of the end portions 42a is pulled, and the one end portion 42a moves toward the proximal end 21a of the arm 21. With this, the route length of one of the pair of third routes R3 of the second bending joint 28 becomes short, the one third route R3 being a route through which the part of the second bending joint operating cable 42 which part extends from the intermediate portion to the one end portion 42a extends. Thus, the second bending joint 28 performs the bending operation of bending toward the one third route R3. Further, a part of the second bending joint operating cable 42 which part extends from the intermediate portion to the other end portion 42a is sent out to be sent into the other third route R3 which is increased in the route length out of the pair of third routes R3.

On the other hand, when the intermediate portion of the second bending joint operating cable 42 is moved toward the other side in the extending direction of the second bending joint operating cable 42 by the driving force of the driving portion 3, the part of the second bending joint operating cable 42 which part extends from the intermediate portion to the other end portion 42a is pulled, and the other end portion 42a moves toward the proximal end 21a of the arm 21. With this, the route length of the other of the pair of third routes R3 of the second bending joint 28 becomes short, the other third route R3 being a route through which the part of the second bending joint operating cable 42 which part extends from the intermediate portion to the other end portion 42a extends. Thus, the second bending joint 28 performs the bending operation of bending toward the other third route R3. Further, the part of the second bending joint operating cable 42 which part extends from the intermediate portion to the one end portion 42a is sent out to be sent into the one third route R3 which is increased in the route length out of the pair of third routes R3.

As described above, the distal end 43a of the forceps operating cable (end effector operating cable) 43 is attached to the forceps 22. A part of the forceps operating cable 43 which part extends from the distal end 43a to the proximal end extends through the through hole 30a (see Fig. 6) of the wrist joint 30 and an internal space of the torque transmission tube 44 (i.e., the internal spaces of the joint portion 26 and the flexible shaft 25), and the proximal end of the forceps operating cable 43 is located in the internal space of the base 23. To be specific, the forceps operating cable 43 is inserted through the torque transmission tube 44.

The forceps operating cable operating portion is a mechanism provided in the base 23 and configured to pull the proximal end of the forceps operating cable 43 in an axial direction of the arm 21 by the driving force of the driving portion 3, the proximal end being located in the internal space of the base 23. When the proximal end of the forceps operating cable 43 is pulled by the driving force of the driving portion 3, the forceps operating cable 43 moves relative to the arm 21 in an extending direction of the forceps operating cable 43, and as a result, the forceps 22 operates.

Fig. 6 is a partial breakaway view showing a configuration example of the wrist joint 30.

The torque transmission tube 44 has flexibility and is formed in a tubular shape. The torque transmission tube 44 can transmit torque, applied to the proximal end thereof, to the distal end 44b directed in an arbitrary direction. To be specific, the torque transmission tube 44 is configured such that by rotating the proximal end, the distal end 44b is rotated in accordance with a rotation amount of the proximal end through an intermediate portion thereof bent in an arbitrary shape. As shown in Fig. 7, the distal end 44b of the torque transmission tube 44 is fixed to a peripheral portion of the through hole 30a of the wrist joint 30. Apart of the torque transmission tube 44 which part extends from the distal end 44b to the proximal end extends through an internal space of the first route R1 of the second bending joint 28, the internal space of the connecting portion 29, an internal space of the first route R1 of the first bending joint 27, and the internal space of the flexible shaft 25, and the proximal end of the torque transmission tube 44 is located in the internal space of the base 23.

The torque transmission tube rotating portion is a mechanism provided in the base 23 and configured to rotate the proximal end of the torque transmission tube 44 by the driving force of the driving portion 3. When the proximal end of the torque transmission tube 44 rotates, the distal end 44b of the torque transmission tube 44 is rotated, and this rotates the wrist joint 30.

The forceps operating cable 43 extends from the forceps 22 through the through hole 30a of the wrist joint 30 to be inserted into the internal space of the torque transmission tube 44 through the distal end 44b of the torque transmission tube 44. The forceps operating cable 43 further extends through the internal space of the torque transmission tube 44 to the base 23. In the internal space of the base 23, the forceps operating cable 43 is led out from the proximal end of the torque transmission tube 44 to an outside of the torque transmission tube 44. Therefore, the forceps operating cable 43 extends along a central axis of the torque transmission tube 44 or its vicinity. The route length from the proximal end of the torque transmission tube 44 to the distal end 44a along the central axis hardly changes between when the arm 21 is stretched and when the arm 21 is bent. Therefore, it is possible to prevent a case where by bending the arm 21, the forceps operating cable 43 is moved in the extending direction, and this operates the forceps 22. Further, it is possible to prevent a case where the forceps operating cable 43 contacts the first bending joint operating cable 41 and the second bending joint operating cable 42 and also possible to prevent a case where when the first bending joint operating cable 41 or the second bending joint operating cable 42 operates, the forceps operating cable 43 unexpectedly operates.

The first bending joint operating cable 41 and the second bending joint operating cable 42 extend through a space between the flexible shaft 25 and the torque transmission tube 44 to reach the base 23. Therefore, the operations of the first bending joint operating cable 41 and the second bending joint operating cable 42 and the operations of the forceps operating cable 43 and the torque transmission tube 44 can be separated from each other. On this account, the first bending joint 27 and the second bending joint 28 can be operated independently from the operations of the forceps 22 and the wrist joint 30.

Since the torque transmission tube 44 has flexibility, the torque transmission tube 44 is bendable together with the flexible shaft 25.

As shown in Fig. 2, the guide pipe 6 is a flexible tubular body, and the flexible shaft 25 is inserted through the guide pipe 6. In a use state shown in Fig. 2, the distal end 25b of the flexible shaft 25 projects from a distal end 6b of the guide pipe 6. Therefore, the joint portion 26 and the forceps 22 project from the distal end 6b of the guide pipe 6. A length of the guide pipe 6 is shorter than a length of the flexible shaft 25. Further, the guide pipe 6 is formed to have a size through which the flexible shaft 25, the joint portion 26, and the forceps 22 can be inserted. Therefore, by inserting the forceps 22, the joint portion 26, and the flexible shaft 25 into the guide pipe 6 through a proximal end 6a of the guide pipe 6 and sending the distal end of the robot main body 2 into the guide pipe 6, the distal end of the robot main body 2 can be sent into the guide pipe 6 toward the distal end 6b, and the forceps 22, the joint portion 26, and the distal end 25b of the flexible shaft 25 can project from the distal end 6b of the guide pipe 6. The guide pipe 6 is configured such that: the inserted surgical robot 1 and the inserted endoscope 101 can be smoothly moved in an extending direction of the guide pipe 6; and the inserted surgical robot 1 and the inserted endoscope 101 can be smoothly rotated around an axis of the guide pipe 6.

In the present embodiment, the guide pipe 6 is formed separately from the collectively bundling pipe 102. However, the guide pipe 6 may be formed integrally with the collectively bundling pipe 102.

The guide pipe 6 includes a distal end curving mechanism configured to curve the distal end 6b. The distal end curving mechanism can curve the distal end 6b in a predetermined direction by operating an operating portion (not shown). To be specific, the guide pipe 6 is configured to be bendable such that the distal end 6b of the guide pipe 6 is directed in a direction perpendicular to the axis of the guide pipe 6 in a virtual plane including the axis of the guide pipe 6. Therefore, when the distal end curving mechanism curves the distal end 6b of the guide pipe 6, the flexible shaft 25 inserted through an internal space of the guide pipe 6 curves, and thus, the first bending joint operating cable 41, the second bending joint operating cable 42, the torque transmission tube 44, and the forceps operating cable 43, which are inserted through the internal space of the flexible shaft 25, curve. As described above, the arm 21 is configured to curve also by the first bending joint 27 and the second bending joint 28. Therefore, the arm 21 of the robot main body 2 is configured to curve at three positions in the vicinity of an affected part. Thus, operability of the robot main body 2 can be improved.

Further, rigidity of the guide pipe 6 in the bending direction is higher than the rigidity of the flexible shaft 25 in the bending direction. With this, it is possible to prevent a case where the guide pipe 6 deforms when the flexible shaft 25 is rotated around the axis thereof. Thus, an angular position of the flexible shaft 25 can be changed while maintaining an extending direction of the flexible shaft 25.

### Configuration Example of Driving Portion

As shown in Fig. 2, the driving portion 3 includes the robot main body driving mechanism 51 configured to drive the robot main body 2.

As shown in Fig. 6, the robot main body driving mechanism 51 includes a first bending joint driving portion 35, a second bending joint driving portion 36, a wrist joint driving portion 38, and a forceps driving portion (end effector driving portion) 37. Each of the first bending joint driving portion 35, the second bending joint driving portion 36, the wrist joint driving portion 38, and the forceps driving portion 37 includes, for example, a servo motor.

By attaching the base 23 to the robot main body driving mechanism 51, the first bending joint driving portion 35 is connected to the first bending joint operating cable 41. The first bending joint operating cable 41 is moved (reciprocated) in the extending direction of the first bending joint operating cable 41 by the driving force of the first bending joint driving portion 35. With this, the first bending joint 27 performs the bending operation.

By attaching the base 23 to the robot main body driving mechanism 51, the second bending joint driving portion 36 is connected to the second bending joint operating cable 42. The second bending joint operating cable 42 is moved (reciprocated) in the extending direction of the second bending joint operating cable 42 by the driving force of the second bending joint driving portion 36. With this, the second bending joint 28 performs the bending operation.

By attaching the base 23 to the robot main body driving mechanism 51, the wrist joint driving portion 38 is connected to the torque transmission tube 44. The proximal end of the torque transmission tube 44 is rotated by the driving force of the wrist joint driving portion 38. With this, the wrist joint 30 is rotated, and the forceps 22 is rotated around the axis L.

By attaching the base 23 to the robot main body driving mechanism 51, the forceps driving portion 37 is connected to the forceps operating cable 43. The forceps operating cable 43 is moved (reciprocated) in the extending direction of the forceps operating cable 43 by the driving force of the forceps driving portion 37. With this, the forceps 22 opens or closes.

### Configuration Examples of Control Unit and Operating Portion

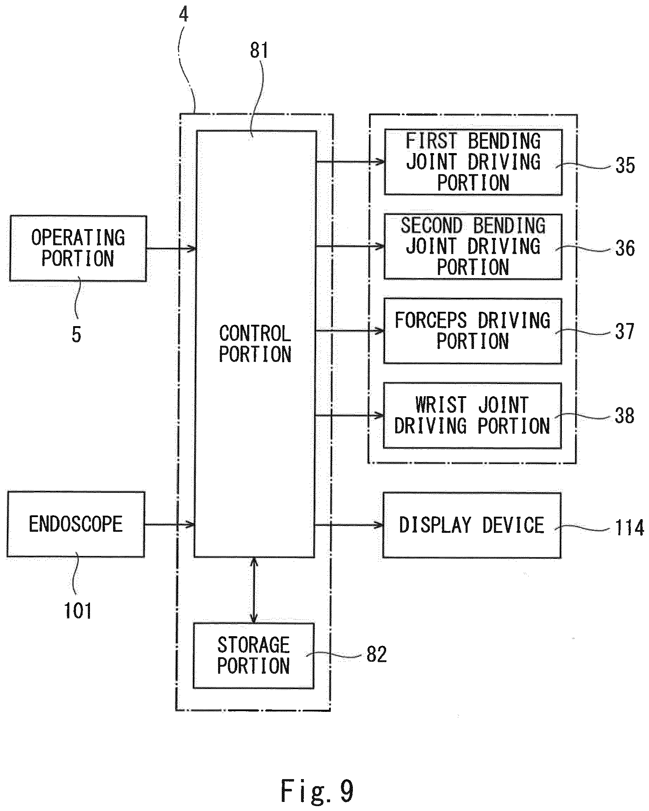

Fig. 9 is a block diagram showing a configuration example of the control unit 4.

The control unit 4 included in the robot main body 2 includes: a control portion 81 including a calculation unit, such as a CPU; and a storage portion 82 including a memory, such as ROM or RAM. The control portion 81 may be constituted by a single control unit which performs centralized control or may be constituted by a plurality of control units which cooperate to perform distributed control. Based on data received from the operating portion 5, the control portion 81 controls an operation of the robot main body driving mechanism 51 of the surgical robot 1 to control an operation of the surgical robot 1. Further, the control portion 81 processes image data, received from the endoscope 101, to transmit the processed image data to the display device 114. The storage portion 82 stores predetermined control programs, and the control portion 81 reads out and executes the control programs to control the operation of the surgical robot 1.

The operator W operates the operating portion 5 to input an operation instruction to be executed by the surgical robot 1. The operating portion 5 is configured to be communicable with the control portion 81. The operating portion 5 converts the operation instruction to be executed by the surgical robot 1 into data to transmit the data to the control portion 81, the operation instruction being input by the operator W.

### Example of Use

Next, an example of use of the surgical robot 1 will be explained.

First, as shown in Fig. 2, one or more guide pipes 6 are inserted into the collectively bundling pipe 102 through an opening of a proximal end 102a of the collectively bundling pipe 102 and are sent into the collectively bundling pipe 102 until the distal end 6b of the guide pipe 6 projects from a distal end 102b of the collectively bundling pipe 102. Similarly, the endoscope 101 is sent into the collectively bundling pipe 102 until a distal end of the endoscope 101 projects from the distal end 102b of the collectively bundling pipe 102.

Next, a trocar 110 is placed on a part of a body surface of the patient P, one or more surgical robots 1 and the endoscope 101 being inserted into the part of the body surface.

Next, the collectively bundling pipe 102 is inserted into the trocar 110 placed on the body surface of the patient P. Then, the inside of the body of the patient P is visually recognized by the endoscope 101, and the distal end 102b of the collectively bundling pipe 102 is located in the vicinity of a treated part of the patient P. The collectively bundling pipe 102, the endoscope 101, and the guide pipe 6 have flexibility. Therefore, for example, even when an organ of the patient P is located on a virtual straight line passing through the part on which the trocar 110 is placed and the treated part, the collectively bundling pipe 102, the endoscope 101, and the guide pipe 6 can be curved to bypass the organ. Thus, the distal end 102b of the collectively bundling pipe 102 can be introduced to the vicinity of the treated part.

Next, the arm 21 of the robot main body 2 of each of the one or more surgical robots 1 is inserted into the guide pipe 6 through an opening of the proximal end 6a of the guide pipe 6 and is sent into the guide pipe 6 until the distal end 25b of the flexible shaft 25 projects from the distal end 6b of the guide pipe 6. With this, the one or more surgical robots 1 and the endoscope 101 can be collectively bundled by the collectively bundling pipe 102 to be integrally introduced to the vicinity of the treated part of the patient P.

As described above, the guide pipe 6, the first bending joint 27, the second bending joint 28, the flexible shaft 25, the first bending joint operating cable 41, the second bending joint operating cable 42, the torque transmission tube 44, and the forceps operating cable 43 are configured to be bendable. Therefore, even when the collectively bundling pipe 102 introduced into the body of the patient P is being curved, the guide pipe 6 and the arm 21 can be sent into the collectively bundling pipe 102 to curve along the curved route in the collectively bundling pipe 102, and the guide pipe 6 and the distal end 21b of the arm 21 can project from the distal end 102b of the collectively bundling pipe 102.

Next, the base 23 is attached to the robot main body driving mechanism 51, and with this, the driving force transmission mechanism 24 of the robot main body 2 and the robot main body driving mechanism 51 are coupled to each other. Thus, the driving force of the robot main body driving mechanism 51 is transmitted to the first bending joint 27, the second bending joint 28, the forceps 22, and the wrist joint 30 through the first bending joint operating cable operating portion, second bending joint operating cable operating portion, forceps operating cable operating portion, and torque transmission tube rotating portion of the driving force transmission mechanism 24.

Next, the distal end 6b of the guide pipe 6 is curved by operating the operating portion of the distal end curving mechanism of the guide pipe 6 of each surgical robot 1. With this, the distal end 21b of the arm 21 can be moved in a direction perpendicular to an axis of the distal end 102b of the collectively bundling pipe 102, and the distal ends 21b of the arms 21 of the surgical robots 1 can be moved away from each other. Therefore, it is possible to prevent a case where the distal ends 21b of the arms 21 of the surgical robots 1 interfere with each other during the operations of the surgical robots 1. Thus, the operability of the surgical robot 1 can be improved.

Next, the operator W operates the operating portion 5 while confirming the image taken by the video camera of the endoscope 101 and displayed on the display device 114. Then, based on the data received from the operating portion 5, the control portion 81 controls the operation of the robot main body driving mechanism 51 to control the operation of the surgical robot 1.

At this time, when the control portion 81 determines that the operation instruction to be executed by the surgical robot 1 contains an operation instruction of bending the first bending joint 27, the control portion 81 drives the robot main body driving mechanism 51 to bend the first bending joint 27. With this, the forceps 22 moves in the bending direction of the first bending joint 27.

Further, when the control portion 81 determines that the operation instruction to be executed by the surgical robot 1 contains an operation instruction of bending the second bending joint 28, the control portion 81 drives the robot main body driving mechanism 51 to bend the second bending joint 28. With this, the forceps 22 moves in the bending direction of the second bending joint 28.

Further, when the control portion 81 determines that the operation instruction to be executed by the surgical robot 1 contains an operation instruction of causing the forceps 22 to perform the holding operation or the releasing operation, the control portion 81 drives the robot main body driving mechanism 51 to cause the forceps 22 to perform the holding operation or the releasing operation.

Further, when the control portion 81 determines that the operation instruction to be executed by the surgical robot 1 contains an operation instruction of rotating the wrist joint 30, the control portion 81 drives the robot main body driving mechanism 51 to rotate the wrist joint 30. As above, the wrist joint 30 is rotated by the rotation of the torque transmission tube 44 which can rotate the distal end 44b in accordance with a rotation amount of the proximal end thereof through the intermediate portion thereof bent in an arbitrary shape. Therefore, even when the flexible shaft 25 and the torque transmission tube 44 are in a bent state, or even while bending the flexible shaft 25 and the torque transmission tube 44, the wrist joint 30 can be accurately rotated.

Then, the proximal end 21a of the arm 21 of the robot main body 2 is further inserted into the guide pipe 6, and this increases a projection amount of the distal end 25b of the flexible shaft 25 from the distal end 6b of the guide pipe 6. With this, the forceps 22 can be moved close to the treated part. Further, the proximal end 21a of the arm 21 of the robot main body 2 is pulled out from the guide pipe 6, and this decreases the projection amount of the distal end 25b of the flexible shaft 25 from the distal end 6b of the guide pipe 6. With this, the forceps 22 can be moved away from the treated part. As described above, the flexible shaft 25 has high rigidity in the axial direction. Therefore, by moving the proximal end 21a of the arm 21 in the axial direction, the distal end 25b of the flexible shaft 25 can be moved in the axial direction in accordance with the movement of the proximal end 21a.

It should be noted that the arm 21 may be inserted into and pulled out from the guide pipe 6 by, for example, moving the robot main body 2 in the axial direction of the proximal end 25a of the flexible shaft 25.

The projection amount of the distal end 25b of the flexible shaft 25 from the distal end 6b of the guide pipe 6 is set within, for example, a range of not less than 0 mm and not more than 30 mm. With this, an error between the position of the forceps 22 and a target position due to the slackening of the flexible shaft 25 can be suppressed, and a movable range of the forceps 22 can be increased. Thus, the operability of the surgical robot 1 can be improved.

As explained above, the surgical robot 1 of the present invention can bend the arm 21 by the first bending joint 27 and the second bending joint 28, which are continuous with the distal end 25b of the flexible shaft 25, to change the direction of the forceps 22. Therefore, the forceps 22 can be directed in a target direction in the vicinity of the treated part. Further, the positions of the first bending joint 27 and the second bending joint 28 relative to the forceps 22 in the extending direction of the arm 21 do not change. Therefore, the operator W can easily predict how the forceps 22 moves by the bending of the first bending joint 27 or the second bending joint 28.

Further, an angular position of the forceps 22 directed in the target direction can be adjusted around an axis of the forceps 22 by rotating the wrist joint 30.

The wrist joint 30 is rotated by rotating the torque transmission tube 44. Therefore, it is possible to prevent a case where the wrist joint 30 moves when the flexible shaft 25, the first bending joint 27, and the second bending joint 28 are bent. To be specific, for example, in some cases, if the wrist joint 30 is configured to be operated in such a manner that a cable inserted into the flexible shaft 25 is operated in an extending direction of the cable, and when the flexible shaft 25, the first bending joint 27, and the second bending joint 28 are bent, the route length of the internal spaces of the flexible shaft 25, the first bending joint 27, and the second bending joint 28 changes, and a distal end of the cable is operated by this change, and as a result, the wrist joint 30 unexpectedly operates. According to the surgical robot 1 of the present invention, even when the flexible shaft 25, the first bending joint 27, and the second bending joint 28 are bent, the torque transmission tube 44 does not rotate around the axis thereof. Therefore, it is possible to prevent a case where the wrist joint 30 unexpectedly operates when the flexible shaft 25, the first bending joint 27, and the second bending joint 28 are bent.

Thus, the operability of the surgical robot 1 can be improved.

From the foregoing explanation, many modifications and other embodiments of the present invention are obvious to one skilled in the art. Therefore, the foregoing explanation should be interpreted only as an example and is provided for the purpose of teaching the best mode for carrying out the present invention to one skilled in the art. The structures and/or functional details may be substantially modified within the scope of the present invention.

### Reference Signs List

- 1: surgical robot
- 2: robot main body
- 3: driving portion
- 4: control unit
- 5: operating portion
- 6: guide pipe
- 21: arm
- 22: forceps
- 23: base
- 24: driving force transmission mechanism
- 25: flexible shaft
- 26: joint portion
- 27: first bending joint
- 28: second bending joint
- 29: connecting portion
- 30: wrist joint
- 31: frame member
- 32: operating cable coupling portion
- 35: first bending joint driving portion
- 36: second bending joint driving portion
- 37: forceps driving portion
- 38: wrist joint driving portion
- 41: first bending joint operating cable
- 42: second bending joint operating cable
- 43: forceps operating cable
- 44: torque transmission tube
- 51: robot main body driving mechanism
- 81: control portion
- 82: storage portion
- 100: surgical robot system
- 101: endoscope
- 102: collectively bundling pipe
- 110: trocar
- 111: operating table
- 113: surgical robot support base
- 114: display device

## Claims

1. A surgical robot comprising:
a robot main body including
a hollow flexible shaft having flexibility,
a hollow bending joint including a proximal end continuous with a distal end of the flexible shaft, the bending joint being bendable in a direction perpendicular to an axis of the bending joint,
a wrist joint continuous with a distal end of the bending joint and rotatable around an axis of the wrist joint,
an end effector attached to the wrist joint,
a hollow torque transmission tube having flexibility, inserted through the flexible shaft and the bending joint, and including a distal end attached to the wrist joint, and
a bending joint operating cable inserted through the flexible shaft and including a distal end attached to the bending joint, the bending joint being configured to perform a bending operation by operating the bending joint operating cable in an extending direction of the bending joint operating cable;
a guide pipe having flexibility, the flexible shaft being inserted through the guide pipe; and
a robot main body driving mechanism including
a bending joint driving portion configured to operate the bending joint operating cable in the extending direction of the bending joint operating cable by driving force of the bending joint driving portion and
a wrist joint driving portion configured to rotate a proximal end of the torque transmission tube by driving force of the wrist joint driving portion.

2. The surgical robot according to claim 1, wherein the guide pipe includes a distal end curving mechanism configured to curve a distal end of the guide pipe.

3. The surgical robot according to claim 1 or 2, wherein rigidity of the guide pipe in a bending direction is higher than rigidity of the flexible shaft in the bending direction.

4. The surgical robot according to any one of claims 1 to 3, wherein:
the robot main body includes an end effector operating cable inserted through the torque transmission tube and including a distal end attached to the end effector;
the end effector is configured to be operated by operating the end effector operating cable in an extending direction of the end effector operating cable; and
the robot main body driving mechanism includes an end effector driving portion configured to operate the end effector operating cable in the extending direction of the end effector operating cable by driving force of the end effector driving portion.
